Europäisches Patentamt

European Patent Office

Office européen des brevets

(11) Publication number: **0 143 641**
**B1**

# EUROPEAN PATENT SPECIFICATION

(45) Date of publication of patent specification: **07.09.88**

(51) Int. Cl.⁴: **A 61 B 17/06**

(21) Application number: **84308203.3**

(22) Date of filing: **27.11.84**

(54) Unitary armed suture mounting board.

(30) Priority: **28.11.83 US 555724**

(43) Date of publication of application:
**05.06.85 Bulletin 85/23**

(45) Publication of the grant of the patent:
**07.09.88 Bulletin 88/36**

(84) Designated Contracting States:
**AT BE CH DE FR GB IT LI NL SE**

(56) References cited:
**US-A-4 183 431**
**US-A-4 287 987**

(73) Proprietor: **ETHICON INC.**
**U.S. Route 22**
**Somerville New Jersey 08876 (US)**

(72) Inventor: **Roshdy, Constance Elizabeth**
**500 Adams Lane, Apt. 1-I**
**North Brunswick, N.Y. 08902 (US)**

(74) Representative: **Jones, Alan John et al**
**CARPMAELS & RANSFORD 43 Bloomsbury**
**Square**
**London, WC1A 2RA (GB)**

Courier Press, Leamington Spa, England.

## Description

The invention relates to an element for mounting armed sutures, and more particularly, to a package utilizing the same.

Packages for surgical sutures are designed and constructed according to the nature of the suture and its intended use. The ideal package holds and protects the suture during handling and storage, and still allows the suture to be readily removed with a minimum of handling that could impair the suture.

Very fine sutures, such as those that are intended for use in ophthalmic surgery, have special requirements owing to the very fine size of the needles and small diameter of the suture strands, often as small or smaller than a human hair. For instance, while it is desirable to hold the suture firmly in place in the package to prevent entangling of the strand, it is also necessary to be able to remove the suture without damage to the needle or without fraying or weakening the strand.

The present invention is directed to a suture mounting element and to a package utilizing the same that is particularly adapted for use with very fine size sutures.

In US—A—4,120,395, there is disclosed a package for double-armed sutures, particularly ophthalmic sutures, comprising a one-piece folded packet having needle mounting means and suture loop retaining means which are readily accessible when the package is opened. Both the needle mounting means and the loop retaining means can be pieces of soft polymeric foam having slits therein.

US—A—4,135,623 shows a package for double-armed sutures.

US—A—4,287,987 discloses an armed suture holder, particularly for armed opthalmic sutures. This patent shows an element for holding a needled suture, said suture comprising a suture strand having at least one end attached to a needle and having a first portion of said strand remote from the end attached to said needle and a second portion of said strand intermediate said first portion and the end attached to said needle, said element comprising a unitary piece of soft flexible polymeric foam, said element having a longitudinal medial axis, a peripheral wall, first and second sides and first and second ends, said element further comprising:

a first peripheral wall portion of said first side a first predetermined distance away from said longitudinal medial axis;

a second peripheral wall portion of said first side at or near said first end, said second peripheral wall portion being a second predetermined distance from said longitudinal media axis that is less than said first predetermined distance away from said longitudinal medial axis, said second peripheral portion thereby comprising a cut out portion of said first side;

a first retaining means for retaining said needle, said first retaining means being in said second peripheral wall portion;

a second retaining means spaced from said first retaining means for retaining said first portion of said strand, said second retaining means being at or near said first end; and

securing means remote from said first and second retaining means.

The element according to the present invention, for holding a needled suture, is characterised in that:

said securing means is an arcuate slit within the body of said element for frictionally engaging said second portion of said suture strand when said strand is inserted into said arcuate slit and is extended relatively tautly from said first retaining means via said arcuate slit to said second retaining means, and for retaining said strand entirely on the surface of said element so that is does not cross itself at any point.

The present invention also provides a suture package which utilizes the above-described element of the present invention.

Some embodiments of the present invention are now described, by way of example only, with reference to the accompanying drawings, in which:

Fig. 1 is a perspective drawing of a suture package illustrating a preferred embodiment of the invention;

Fig. 2 is an exploded view of the package of Fig. 1;

Fig. 3 is a perspective view of the suture mounting element shown in Fig. 1, shown with a different length suture,

Fig. 4 is a view similar to Fig. 3 wherein a still different length suture is shown mounted thereon;

Fig. 5 shows a double armed suture folded in half, ready to be mounted on an element in accordance with the invention.

As used herein, the term "suture" means elongated, thread-like strands suitable for suturing, ligating, or other surgical procedures, with or without needles attached. A "single-armed suture" is a suture having a needle affixed to one end, and the term "double-armed suture" refers to a suture having needles attached to both ends. The term "suture strand" refers specifically to the elongated thread-like portion of the suture.

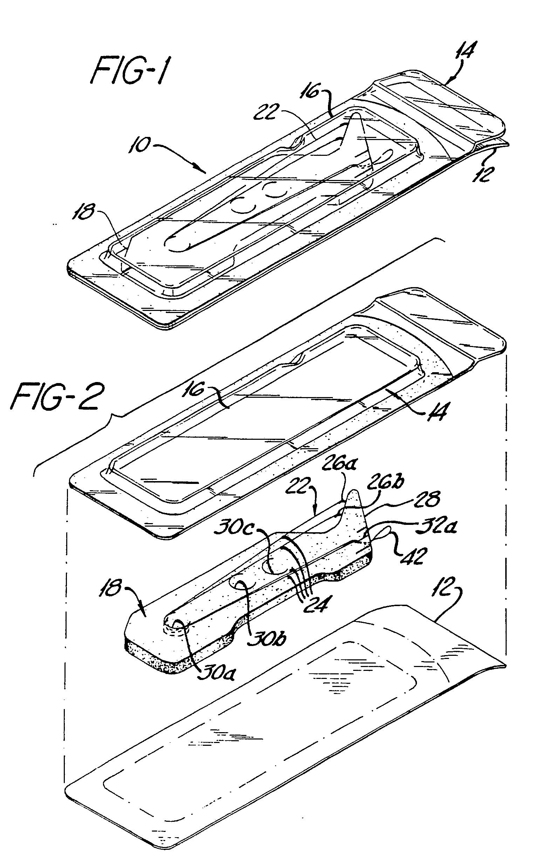

Referring now to the drawings, Figs. 1 and 2 show a suture package according to the invention. The package 10 contains a rear panel 12 bonded to a front panel 14, which is preferably made of clear plastic. The rear panel 12 may be made of a material such as spunbonded polyolefin that is pervious to sterilizing gas, to facilitate sterilization of the package. The front panel 14 has a blister 16 molded in it which, together with the rear panel 12, defines a suitable storage compartment. Within the suture storage compartment there is a mounting board 18 made from a single piece of soft, flexible, polymeric foam, such as polyethylene foam, which, in an alternative embodiment, may be bonded to a paperboard backing, which acts as a stiffening element. Mounted on the mounting board 18 is a double-armed suture 22 comprising a strand 24 and two needles 26a, 26b.

The suture 22 is mounted on the mounting board 18 in the following manner:

The points of the two needles 26a, 26b are inserted in the foam at one end of the mounting board 18, as is shown in the Figures. The doubled suture strand 24 is held relatively tautly so that the strand 24 is held under slight tension from the mounted needles 26a, 26b, and the tautly held strand 24 is pressed into a round slit 30a that is cut in the foam board 18. The strand 24 is then held under slight tension from the slit 30a, and the end 42 of the doubled strand 24 is inserted in a slit 32a at the same end 28 of the board 18 at which the needles 26a, 26b are inserted, but on the opposite side. By mounting the suture 22 in this manner, it is held firmly in a protected manner so that it is easy for the surgeon to remove the suture 22 from the board 18. The invention is particularly useful for packaging and storing very fine sutures as those that are used in ophthalmic surgery because such fine sutures are held firmly in place, and do not tend to "float", until the surgeon is ready to use them.

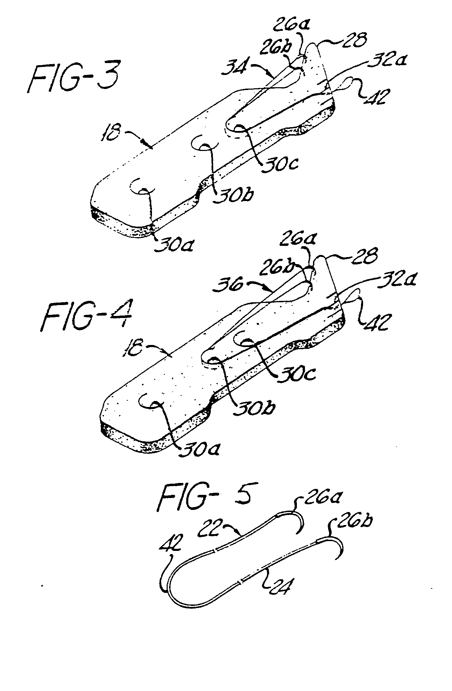

In Figs. 3 and 4 there are shown sutures 34, 36 having different lengths than the suture 22 shown in Figs. 1 and 2, and which are mounted on the mounting board 18 in accordance with the invention.

The invention is useful for packaging both single-armed and double-armed sutures, although its use with very fine double-armed sutures is preferred.

The invention has the virtue of simplicity of design and operation. It also has the advantage of visually presenting the suture to the surgeon before the package is opened, so the surgeon can make sure that the correct size and length suture is contained therein before the sterile package is opened.

## Claims

1. An element (18) for holding a needled suture (22), said suture comprising a suture strand (24) having at least one end attached to a needle (26a, 26b) and having a first portion (42) of said strand (24) remote from the end attached to said needle (26a, 26b) and a second portion of said strand intermediate said first portion (42) and the end attached to said needle (26a, 26b), said element (18) comprising a unitary piece of soft flexible polymeric foam, said element having a surface, a longitudinal medial axis, a peripheral wall, first and second sides and first (28) and second (38) ends, said element (18) further comprising:

(a) a first peripheral wall portion of said first side a first predetermined distance away from said longitudinal medial axis;

(b) a second peripheral wall portion of said first side at or near said first end (28), said second peripheral wall portion being a second predetermined distance from said longitudinal medial axis that is less than said first predetermined distance away from said longitudinal medial axis, said second peripheral portion thereby comprising a cut out portion of said first side;

(c) a first retaining means for retaining said needle, (26a, 26b), said first retaining means being in said second peripheral wall portion;

(d) a second retaining means (32a) spaced from said first retaining means for retaining said first portion (42) of said strand (24), said second retaining means being at or near said first end (28); and

(e) securing means (30a, 30b, 30c) remote from said first and second (32a) retaining means;

characterised in that;

said securing means is an arcuate slit (30a, 30b, 30c) within the body of said element (18) for frictionally engaging said second portion of said suture strand (24) when said strand (24) is inserted into said arcuate slit and is extended relatively tautly from said first retaining means via said arcuate slit to said second retaining means (32a), and for retaining said strand (24) entirely on the surface of said element so that it does not cross itself at any point.

2. The element of claim 1 wherein said first retaining means comprises said soft, flexible polymeric foam, for receiving therein the point of said needle.

3. The element of claim 1 or claim 2 wherein said second retaining means comprises a slit (32a) in said foam.

4. The element of any one of claims 1 to 3 adapted for retaining a double-armed suture.

5. A suture package comprising a rear panel (12) bonded to a front panel (14) and containing a needled suture (22) mounted on the element (18) of any one of claims 1 to 4.

6. The suture package of claim 5 in sterile condition.

## Patentansprüche

1. Ein Element (18), welches als Träger für ein mit einer Nadel versehenes, chirurgisches Nahtmaterial (22) dient, wobei das genannte Nahtmaterial ein Fadenmaterial (24) des Nahtmaterials umfaßt, welches Fadenmaterial mit wenigstens einem Ende an einer Nadel (26a, 26b) befestigt ist, und wobei das Nahtmaterial einen ersten Teil (42) des genannten Fadenmaterials (24) aufweist, der entfernt von dem Ende gelegen ist, das an der genannten Nadel (26a, 26b) befestigt ist, und wobei das Nahtmaterial einen zweiten Teil des genannten Fadenmaterials aufweist, welcher zwischen dem genannten ersten Teil (42) und dem an der genannten Nadel (26a, 26b) befestigten Ende gelegen ist, wobei das genannte Element (18) ein einheitliches Stück eines weichen, biegsamen Polymerschaumes umfaßt, und wobei das genannte Element eine Oberfläche, eine in der Längsrichtung verlaufende Mittelachse, eine Umfangswand, eine erste und eine zweite Seite, und ein erstes Ende (28) sowie ein zweites Ende (38) aufweist, und wobei das genannte Element (18) weiterhin umfaßt:

(a) einen ersten Umfangswandteil der genannten ersten Seite, der um einen ersten, vorbestimmten Abstand von der genannten, in der Längsrichtung verlaufenden Mittelachse entfernt angeordnet ist;

(b) einen zweiten Umfangswandteil der genannten ersten Seite, der an dem genannten ersten Ende (28) oder in der Nähe desselben angeordnet ist, wobei sich der genannte zweite Umfangswandteil um einen zweiten vorbestimmten Abstand von der genannten, in der Längsrichtung verlaufenden Mittelachse entfernt befindet, und wobei dieser zweite vorbestimmte Abstand von der genannten, in der Längsrichtung verlaufenden Mittelachse weniger weit entfernt ist als der genannte erste vorbestimmte Abstand, und wobei der genannte zweite Umfangswandteil dadurch einen ausgeschnittenen Teil der genannten ersten Seite umfaßt;

(c) eine erste Halterungseinrichtung zum Festhalten der genannten Nadel (26a, 26b), wobei sich die genannte erste Halterungseinrichtung in dem genannten zweiten Umfangswandteil befindet;

(d) eine zweite Halterungseinrichtung (32a), welche im Abstand von der genannten ersten Halterungseinrichtung angeordnet ist, und welche dazu dient, den genannten ersten Teil (42) des genannten Fadenmaterials (24) festzuhalten, wobei sich die genannte zweite Halterungseinrichtung an dem genannten ersten Ende (28) oder nahe bei demselben befindet; und

(e) Befestigungseinrichtungen (30a, 30b, 30c), welche entfernt von der genannten ersten Halterungseinrichtung und von der genannten zweiten Halterungseinrichtung (32a) angeordnet sind; dadurch gekennzeichnet, daß die genannte Befestigungseinrichtung ein gekrümmter Schlitz (30a, 30b, 30c) innerhalb des Körpers des genannten Elementes (18) ist, der dazu dient, mit dem genannten zweiten Teil des genannten Fadenmaterials (24) des Nahtmaterials in Reibungseingriff zu kommen, wenn das genannte Fadenmaterial (24) in den genannten gekrümmten Schlitz eingeführt und relativ straff von der genannten ersten Halterungseinrichtung über den genannten gekrümmten Schlitz zu der genannten zweiten Halterungseinrichtung (32a) weitergeführt wird, und der weiterhin dazu dient, das genannte Fadenmaterial (24) zur Gänze auf der Oberfläche des genannten Elementes festzuhalten, sodaß sich das Fadenmaterial an keinem Punkt mit sich selbst überkreuzt.

2. Element nach Anspruch 1, worin die genannte erste Halterungseinrichtung den genannten weichen, biegsamen Polymerschaum umfaßt und dazu dient, die Spitze der genannten Nadel in dem Polymerschaum aufzunehmen.

3. Element nach Anspruch 1 oder Anspruch 2, wobei die genannte zweite Halterungseinrichtung einen Schlitz (32a) in dem genannten Schaum umfaßt.

4. Element nach einem der Ansprüche 1 bis 3, welches geeignet ist, ein an seinen beiden Enden mit einer Nadel versehenes Nahtmaterial festzuhalten.

5. Verpackung für Nahtmaterial, umfassend ein hinteres Blatt (12), welches mit einem vorderen Blatt (14) verbunden ist, wobei die Verpackung ein mit einer Nadel versehenes Nahtmaterial (22) enthält, welches auf dem Element (18) nach einem der Ansprüche 1 bis 4 montiert ist.

6. Verpackung für Nahtmaterial nach Anspruch 5 in einem sterilen Zustand.

## Revendications

1. Un élément (18) pour maintenir une suture (22) à aiguilles, cette suture comprenant un fil de suture (24) dont au moins une extrémité est attachée à une aiguille (26a, 26b) et dont une première partie (42) du fil (24) est distante de l'extrémité attachée à l'aiguille (26a, 26b) et une seconde partie de ce fil est intermédiaire entre la première partie (42) et l'extrémité attachée à l'aiguille (26a, 26b), cet élément (18) comprenant une pièce d'un seul tenant en mousse de polymère flexible et molle, cet élément ayant une surface, un axe médian longitudinal, une paroi périphérique, un premier et un second côtés et une première extrémité (28) et une seconde extrémité (38), cet élément (18) comprenant en outre:

(a) une première partie de paroi périphérique du premier côté située à une première distance prédéterminée de l'axe médian longitudinal;

(b) une seconde partie de paroi périphérique du premier côté située à la première extrémité (28) ou près de la première extrémité, ladite seconde partie de paroi périphérique étant située à une seconde distance prédéterminée de l'axe médian longitudinal, cette distance étant inférieure à la première distance prédéterminée de l'axe médian longitudinal, la seconde partie périphérique comprenant ainsi une partie découpée du premier cote;

(c) un premier dispositif de maintien servant à maintenir l'aiguille (26a, 26b), ce premier dispositif de maintien étant situé dans la seconde partie de paroi périphérique;

(d) un second dispositif de maintien (32a) distant du premier dispositif de maintien, servant à maintenir la première partie (42) du fil (24), le second dispositif de maintien étant situé à la première extrémité (28) ou près de la première extrémité; et

(e) un dispositif d'ancrage (30a, 30b, 30c) éloigné du premier et du second dispositifs de maintien (32a);
caractérisé en ce que:
le dispositif d'ancrage est constitué par une fente arquée (30a, 30b, 30c) au sein du corps de l'élément (18) pour enclencher par frottement la seconde partie du fil de suture (24) lorsque le fil (24) est inséré dans la fente arquée et est allongé de façon relativement tendue depuis le premier dispositif de maintien, par le biais de la fente arquée, jusqu'au second dispositif de maintien (32a), et pour maintenir le fil (24) entièrement sur la surface de l'élément, de sorte qu'il ne se croise en aucun point.

2. L'élément selon la revendication 1, dans lequel le premier dispositif de maintien comprend la mousse de polymère flexible et molle destinée à y recevoir la pointe de l'aiguille.

3. L'élément selon la revendication 1 ou 2, dans lequel le second dispositif de maintien comprend une fente (32a) dans la mousse.

4. L'élément selon l'une quelconque des revendications 1 à 3, adapté pour maintenir une suture avec fil serti d'une aiguille à chaque extrémité.

5. Un empaquetage pour sutures comprenant un panneau arrière (12) collé à un panneau avant (14) et contenant une suture (22) à aiguilles montée sur l'élément (18) selon l'une quelconque des revendications 1 à 4.

6. L'empaquetage pour sutures selon la revendication 5, dans des conditions stériles.

FIG-1

FIG-2

FIG-3

FIG-4

FIG-5